# EUROPEAN PATENT APPLICATION

(11) **EP 4 116 226 A1**
(43) Date of publication of application: **11.01.2023**
(21) Application number: 21822314.7
(22) Date of filing: 07.06.2021
(51) Int. Cl.: B65F 1/06, B65F 1/14, B65F 1/00, B65B 51/10, B65B 61/10, B29C 65/22, B65F 7/00

(54) **HOT MELTING MECHANISM, FUSING MECHANISM, HEATING BASE MECHANISM, CLOSING AND PACKING MECHANISM, STERILIZATION DEVICE, AND INTELLIGENT TRASH CAN**

(30) Priority: 09.06.2020 CN 202010516105; 09.06.2020 CN 202021040149 U; 09.06.2020 CN 202021040492 U; 18.06.2020 CN 202021132164 U; 18.06.2020 CN 202021132168 U; 18.06.2020 CN 202021132795 U
(71) Applicant: Dreame Technology (shanghai) Co., Ltd., Minhang District Shanghai 201100 (CN)
(72) Inventor: GE, Wei, Shanghai 201100 (CN); YU, Qiuhua, Shanghai 201100 (CN); GAO, Shang, Shanghai 201100 (CN)
(74) Representative: dompatent von Kreisler Selting Werner - Partnerschaft von Patent- und Rechtsanwälten mbB
(86) International application number: PCT/CN2021/098619
(87) International publication number: WO 2021/249339

(57) **Abstract**

Disclosed are a hot melting mechanism, a fusing mechanism, a heating base mechanism, a closing and packing mechanism, a sterilization device, and an intelligent trash can. The hot melting mechanism comprises: an insulating base (12) provided with a groove (120) along the length direction; and a hot melting member (11) snap-fitted into the groove (120) and partially exposed of the groove (120). The hot melting member (11) is connected to a power supply circuit by means of a wire (13). In this way, the deformation of a hot melting working section in the length direction is controlled, so that the working edge of the hot melting member is kept straight, and the sealing effect on a garbage bag is thus effectively improved.

## Description

### TECHNICAL FIELD

The present application relates to a technical field of trash cans, and in particular to a hot melting mechanism, a fusing mechanism, a heating seat mechanism, a closing and packing mechanism, a sterilization device and an intelligent trash can.

### BACKGROUND

With the improvement of people's living standards, the amount of garbage generated is also increasing. As a household necessities, trash cans have played an important practical significance in sorting and recycling garbage and keeping the environment clean and tidy. An intelligent trash can includes a microcomputer control unit, an infrared sensor detection device and a mechanical transmission part, which is a new high-tech product integrating machine, light and electricity.

### SUMMARY

In a first aspect, the present application provides a hot melting structure, a fusing mechanism and an intelligent trash can. A slot configured in a hot melting element can limit the position of the hot melting element when the hot melting element is energized and heated, so that the shape of the hot melting element held in the slot in a longitudinal direction is controlled.

A technical solution adopted in the present application is:
a hot melting structure, including: an insulating seat, the insulating seat defining a slot along a length thereof; and a hot melting element held in the slot, the hot melting element being partially exposed in the slot; wherein the hot melting element is connected to a power supply circuit through wires.

In an embodiment of the present application, a cross-section of the hot melting element is rectangular.

In an embodiment of the present application, the hot melting element includes: an intermediate section held in the slot; and a first extension section and a second extension section which are disposed on two sides of the intermediate section, respectively; wherein the first extension section and the second extension section are both connected to the power supply circuit through the wires.

In an embodiment of the present application, an included angle is formed between the first extension section and the intermediate section, and between the second extension section and the intermediate section.

In an embodiment of the present application, the first extension section and the second extension section are inserted into the insulating seat.

In an embodiment of the present application, the wires include: a first wire connected to the first extension section; and a second wire connected to the second extension section.

In an embodiment of the present application, the first extension section and the first wire are connected together by a fixing member, and the second extension section and the second wire are connected together by a fixing member.

In an embodiment of the present application, a lead groove for accommodating the first wire and the second wire is opened on the insulating seat.

In order to solve the above technical problem, another solution proposed by the present application is:
a fusing mechanism, including the aforementioned heat melting structure.

Another solution proposed by the present application is:
an intelligent trash can, including the aforementioned fusing mechanism.

Compared with the prior art, the present application has the following beneficial effects:
in the hot melting structure provided by the present application, a hot-melting working section of the hot melting element is held in the slot of the insulating seat, and the slot can limit the position of the hot-melting working section of the hot melting element. In the above manner, the shape of the hot-melting working section in the longitudinal direction is controlled, so that the working edge of the hot melting element is kept in a straight shape, thereby effectively improving the sealing effect of the garbage bag.

In a second aspect, the present application provides a fusing structure and a trash can having the same.

A technical solution adopted in the present application is:
a fusing structure, including: an insulating seat; a hot melting element disposed on the insulating seat along a length direction of the insulating seat; an insulating elastomer disposed between the insulating seat and the hot melting element; and a pressing rod movably disposed on a side of the hot melting element; wherein the hot melting element is connected to a power supply circuit through wires.

In an embodiment of the present application, the wires are wound on the insulating seat.

In an embodiment of the present application, the hot melting element includes: an intermediate section abutting against the insulating elastomer; and a first extension section and a second extension section which are disposed on two sides of the intermediate section, respectively; wherein the first extension section and the second extension section are both connected to the wires.

In an embodiment of the present application, an included angle is formed between the first extension section and the intermediate section, and between the second extension section and the intermediate section.

In an embodiment of the present application, a first side groove and a second side groove are configured on two sides of the insulating seat, respectively; and the first extension section and the second extension section are received in the first side groove and the second side groove, respectively.

In an embodiment of the present application, the wires include: a first wire connected to the first extension section; and a second wire connected to the second extension section.

In an embodiment of the present application, the first extension section and the first wire are connected together by a fixing member, and the second extension section and the second wire are connected together by a fixing member.

In an embodiment of the present application, a lead groove for accommodating the first wire and the second wire is opened on the insulating seat.

In an embodiment of the present application, the lead groove includes: a first groove for accommodating the first wire; a second groove for accommodating the second wire; and a third groove for accommodating the first wire and the second wire; wherein the first groove and the second groove are disposed along the length direction of the insulating seat; and the third groove is disposed along a height direction of the insulating seat.

Another solution proposed by the present application is:
a trash can, including the aforementioned fusing structure.

Compared with the prior art, the present application has the following beneficial effects:
in the fusing structure provided by the present application, when the pressing rod presses the garbage bag on the hot melting element, the insulating elastomer disposed between the insulating seat and the hot melting element can provide a supporting force to the hot melting element. In the above manner, the garbage bag and the hot melting element can be fully contacted, thereby effectively improving the sealing effect of the garbage bag.

In a third aspect, the present application provides a closing and packing mechanism, and an intelligent trash can, which have the advantages of high space utilization and can effectively expand the area of the garbage throwing port.

A technical solution adopted in the present application is:
a closing and packing mechanism for automatic closing of a garbage bag, including: a pair of first pressing rods, the pair of first pressing rods configured for being synchronously approached or synchronously moved away under the driving of a first belt transmission unit; and a second pressing rod, the second pressing rod being perpendicular to the pair of first pressing rods, and the second pressing rod being configured to perform linear reciprocating motion along a length direction of the pair of first pressing rods under the driving of the second belt transmission unit; wherein the second belt transmission unit includes a pair of second transmission belts which are symmetrically disposed on opposite sides of the pair of first pressing rods and connected with the second pressing rod; and wherein recesses are provided on side walls of the pair of first pressing rods adjacent to the second transmission belts, the recesses are configured such that the second transmission belts can be accommodated in the recesses when the pair of first pressing rods are in an extreme position under a status of being synchronously moved away.

In an embodiment of the present application, a bottom groove wall in the recess is provided with openings distributed along a length direction of the recess, and the openings are configured to form an escape space which can escape a slack side belt body of the second transmission belt.

In an embodiment of the present application, sidewalls of the pair of first pressing rods are formed with through openings extending horizontally therethrough, and the through openings are distributed along the length direction of the pair of first pressing rods; wherein the second pressing rod is disposed through the through openings, and two ends of the second pressing rod are respectively connected to the second transmission belts.

In an embodiment of the present application, the second belt transmission unit further includes a pair of second pulley sets on which the second transmission belts are tensioned, and a second drive motor which drives the pair of second pulley sets to move.

In an embodiment of the present application, each second pulley set includes a second driving wheel and a second driven wheel, both the second driving wheels and the second driven wheels are connected by a transmission shaft; wherein two ends of the pair of first pressing rods are respectively sleeved on the pair of transmission shafts, so as to realize guiding of the pair of first pressing rods.

In an embodiment of the present application, the first belt transmission unit includes a first transmission belt connected with the pair of first pressing rods, a first pulley set tensioned with the first transmission belt, a first drive motor driving the first pulley set to move, and a guiding rod parallel to the pair of first pressing rods and configured for guiding the second pressing rod.

In an embodiment of the present application, an end of the second pressing rod is sleeved on the guiding rod after passing through the second transmission belt.

In an embodiment of the present application, convex wings are provided on the side walls of the pair of first pressing rods away from the second transmission belt, the convex wings are configured to assist in closing a mouth of the garbage bag; wherein the convex wings are horizontally disposed blocks, and a side wall of the convex wing in contact with the garbage bag is convex in a middle and concave on two sides.

In an embodiment of the present application, the convex wings include a first convex wing provided on one of the first pressing rods and a second convex wing provided on a remaining one of the first pressing rods; wherein the first convex wing is located directly above the second convex wing; or the first convex wing is located directly under the second convex wing.

Another solution proposed by the present application is:
an intelligent trash can, including the aforementioned closing and packing mechanism.

Compared with the prior art, the present application has the following beneficial effects:
the closing and packing mechanism, and the intelligent trash can provided by the present application are provided by arranging the recesses on the side walls of the pair of first pressing rods. When the closing and packing mechanism is in a non-working state, that is, when the pair of first pressing rods are in the extreme position under a status of being synchronously moved away, the second transmission belts can be accommodated in the recesses. The present application has the advantage of high space utilization, and can effectively expand the area of the garbage throwing port.

In a fourth aspect, the present application provides a heating seat structure, a closing and fusing mechanism, and an intelligent trash can. The present application can manage and control the thermal expansion deformation of the fusing element due to electricity and heat generation, so as to ensure the position of the intermediate section of the fusing element, so that the working edge of the fusing element can be kept straight.

In order to solve the above-mentioned technical problem, the technical solution adopted in the present application is:
a heating seat structure, applied to a fusing seal of a garbage bag, including: an insulating seat; and a fusing element, the fusing element being in a shape of a sheet and being held in the insulating seat, a side edge of the fusing element extending out of the insulating seat to form a working edge contacting the garbage bag; wherein the fusing element includes a fusing element body of which one side edge is the working edge, and a connecting piece formed on two opposite ends of the fusing element body.

In an embodiment of the present application, the fusing element is a sheet-shaped heating wire having protruding portions at two ends in a longitudinal direction.

In an embodiment of the present application, the insulating seat is provided with a mounting groove; wherein the mounting groove is formed with a notch on a side wall of the insulating seat so that the working edge of the fusing element is exposed.

In an embodiment of the present application, a gap is preset between a side wall of the fusing element located in the mounting groove and a groove wall of the mounting groove, so as to form a deformation space.

In an embodiment of the present application, the insulating seat includes an upper seat body and a lower seat body which are disposed in separate upper and lower parts, the upper seat body and the lower seat body being detachably connected; wherein a bottom of the upper seat body is formed with a pair of positioning posts extending vertically downward, a top of the lower seat body is formed with positioning holes for inserting the positioning posts, and the connecting piece is provided with mounting holes for the positioning posts to pass through.

In an embodiment of the present application, the mounting groove includes a lower groove body formed on the top of the lower seat body and an upper groove body formed at the bottom of the upper seat body; wherein the positioning posts are disposed on the upper groove body, and the positioning holes are disposed in the lower groove body.

In an embodiment of the present application, a pair of limiting posts extending vertically downward are formed at the bottom of the upper seat body, and limiting holes are formed on the top of the lower seat body for inserting the limiting posts.

In an embodiment of the present application, the insulating seat is further provided with a wiring hole which is communicated with the mounting groove.

Another solution proposed by the present application is:
a closing and fusing mechanism, including the aforementioned heating seat structure and a pressing rod assembly configured to drive the garbage bag to be closed to the heating seat structure.

Another solution proposed by the present application is:
an intelligent trash can, including the aforementioned closing and fusing mechanism.

Compared with the prior art, the present application has the following beneficial effects:
in the heating seat structure, the closing and fusing mechanism and the intelligent trash can provided by the present application, the fusing element is in sheet shape and is held in the insulating seat. The thermal expansion and deformation of the fusing element due to electricity and heat can be controlled, so that the position of the intermediate section of the fusing element is ensured. As a result, the working edge of the fusing element can be kept straight, which effectively improves the sealing effect of the garbage bag.

In a fifth aspect, the present application provides a sterilization device and an intelligent trash can, which can effectively prevent the growth of bacteria in the trash can and perform effective sterilization, thereby preventing the user from contacting the bacteria in the trash can.

A technical solution adopted in the present application is:
a sterilization device, applicable to a trash can with a flip cover at a garbage throwing port, the flip cover being pivotally connected to the garbage throwing port to form a closed garbage accommodating space, the sterilization device including: an ultraviolet sterilization lamp detachably disposed on an inner wall of the flip cover to generate ultraviolet rays for sterilization; a fan detachably disposed on the inner wall of the flip cover to circulate the air in the garbage accommodating space; and a cover detachably disposed on the inner wall of the flip cover, and the cover covering an outer periphery of the ultraviolet sterilization lamp and the fan so as to form a sterilization space; wherein the cover is provided with an air inlet and an air outlet, a direction from the air inlet to the air outlet is an air flow direction in the sterilization space, the ultraviolet sterilization lamp is located upstream of the air flow direction, and the fan is located downstream of the air flow direction.

In an embodiment of the present application, the ultraviolet sterilization lamp is disposed adjacent to the air inlet, and the fan is disposed adjacent to the air outlet; and wherein an outlet of the fan abuts against the air outlet.

In an embodiment of the present application, the inner wall of the flip cover is provided with a first snapping structure, the cover is provided with a second snapping structure matched with the first snapping structure; wherein the cover is snap-fitted to the inner wall of the flip cover through cooperation between the second snapping structure and the first snapping structure.

In an embodiment of the present application, the first snapping structure is a hook, and the second snapping structure is a slot matched with the hook; or the first snapping structure is a slot, and the second snapping structure is a hook matched with the slot.

In an embodiment of the present application, the air inlet consists of a plurality of elongated slots distributed in parallel, and the air outlet consists of a plurality of through holes distributed in a circular array.

In an embodiment of the present application, a sealing structure is provided between the inner wall of the flip cover and the cover so as to improve the air tightness of the sterilization space.

In an embodiment of the present application, the sealing structure includes a second annular rib distributed along an edge of the cover and protruding toward the flip cover, and a first annular rib formed on the inner wall of the flip cover and matched with the second annular rib; wherein the second annular rib and the first annular rib are both of an integral annular configuration, and the first annular rib is provided with a sealing groove for inserting the second annular rib.

In an embodiment of the present application, a rib plate is further formed on the inner wall of the flip cover, and the rib plate is configured to enclose a groove to hold the ultraviolet sterilization lamp.

In an embodiment of the present application, both the ultraviolet sterilization lamp and the fan are connected with a power supply circuit board through wires.

Another solution proposed by the present application is:
an intelligent trash can, including: a barrel body, an interior of the barrel body being hollow and a top of the barrel body being opened so as to form a cavity for accommodating garbage and a barrel mouth at a top of the cavity; a barrel cover pivotally connected to the barrel mouth, the barrel cover including a barrel cover body formed with a garbage throwing port and pivotally connected to the barrel mouth; a flip cover pivotally connected to the garbage throwing port; and a sterilization device disposed on the flip cover; wherein the sterilization device is the aforementioned sterilization device.

Compared with the prior art, the present application has the following beneficial effects:
in the sterilization device and the intelligent trash can provided by the present application, the ultraviolet sterilization lamp thereof can generate ultraviolet rays for sterilization. It can effectively prevent the growth of bacteria in the trash can and effectively sterilize. The fan can promote the air circulation in the trash can, effectively improve the sterilization effect, so as to prevent the user from contacting the bacteria in the trash can.

### BRIEF DESCRIPTION OF DRAWINGS

FIG. 1 is a first structural schematic view of a hot melting structure proposed by the present application;
FIG. 2 is a second structural schematic view of the hot melting structure proposed by the present application;
FIG. 3 is an exploded structural schematic view of the hot melting structure in FIG. 1;
FIG. 4 is a cross-sectional structural schematic view of the hot melting element in FIG. 3;
FIG. 5 is a rear structural schematic view of an insulating seat in FIG. 3;
FIG. 6 is a first schematic view of an overall structure of a fusing structure proposed by the present application;
FIG. 7 is a second schematic view of the overall structure of the fusing structure proposed by the present application;
FIG. 8 is a schematic view of an exploded structure of the fusing structure proposed by the present application;
FIG. 9 is a rear structural schematic view of the insulating seat in FIG. 6;
FIG. 10 is a schematic view of a positional relationship between a closing and packing mechanism and a barrel cover proposed by the present application;
FIG. 11 is a schematic view of the closing and packing mechanism proposed by the present application in a top view direction;
FIG. 12 is a schematic view of the closing and packing mechanism proposed by the present application in a bottom view direction;
FIG. 13 is a schematic structural view of the first pressing rod in FIG. 11 from a viewing angle;
FIG. 14 is a schematic structural view of the first pressing rod in FIG. 11 from another viewing angle;
FIG. 15 is a structural schematic view of an intelligent trash can proposed by the present application;
FIG. 16 is a schematic view when the barrel cover in FIG. 15 is in an open state;
FIG. 17 is a schematic structural view of a heating seat structure described in the present application in a front view direction;
FIG. 18 is a structural schematic view of the heating seat structure described in the present application in a rear view direction;
FIG. 19 is an exploded structural schematic view of the heating seat structure described in the present application;
FIG. 20 is a schematic view of a positional relationship between a lower seat body and the fusing element in the present application;
FIG. 21 is a structural schematic view of the lower seat body in the present application;
FIG. 22 is a structural schematic view of the upper seat body in the present application;
FIG. 23 is the structural schematic view of the closing and fusing mechanism described in the present application;
FIG. 24 is a first structural schematic view of an intelligent trash can described in the present application;
FIG. 25 is a second structural schematic view of the intelligent trash can described in the application;
FIG. 26 is a schematic structural view of a traditional heating seat structure;
FIG. 27 is a schematic view of a positional relationship between a sterilization device and a flip cover proposed in the present application;
FIG. 28 is a schematic cross-sectional structural view of the sterilization device proposed by the present application;
FIG. 29 is an exploded schematic view of the sterilization device proposed by the present application in a front view direction;
FIG. 30 is an exploded schematic view of the sterilization device proposed by the present application in a rear view direction;
FIG. 31 is an enlarged structural schematic view of area A in FIG. 29;
FIG. 32 is an enlarged schematic view of region B in FIG. 30;
FIG. 33 is a structural schematic view of the intelligent trash can proposed by the present application; and
FIG. 34 is a schematic view of the positional relationship between a barrel body and the barrel cover of the intelligent trash can proposed in the present application.

### DETAILED DESCRIPTION

The present application will be further described in detail below with reference to the accompanying drawings, so that those skilled in the art can implement it with reference to the description. If there are descriptions involving "first", "second", etc., in the embodiments of the present application, the description of "first", "second", etc., are only used for the purpose of description, and should not be understood as indicating or implying their relative importance or implying the number of technical features indicated. Thus, a feature delimited with "first", "second" may expressly or implicitly include at least one of that feature.

In the description of the present application, it should also be noted that, unless there are more explicit definitions and limitations, the terms "installed", "connected" and "connection" should be understood in a broader sense. For example, the term "connection" may be a fixed connection, a detachable connection, or an integral connection; a mechanical connection or an electrical connection; a direct connection, or an indirect connection through an intermediate medium, or a communication of two elements. For those of ordinary skill in the art, the specific meanings of the above terms in the present application can be understood in specific situations.

In addition, if the meaning of "and/or" appears in the present application, it includes three concurrent solutions. Taking "A and/or B" as an example, it includes a solution A, or a solution B, or a solution that satisfies both the solution A and the solution B. In addition, the technical solutions between the various embodiments can be combined with each other, but must be based on the realization by those of ordinary skill in the art. When a combination of technical solutions contradicts each other or cannot be realized, it shall be considered that such combination of technical solutions does not exist, and is not within the protection scope claimed in the present application.

In order to make the above objects, features and advantages of the present application more clearly understood, the specific embodiments of the present application will be described in detail below with reference to the accompanying drawings. It should be understood that the specific embodiments described herein are only used to explain the present application, but not to limit the present application. In addition, it should be noted that, for the convenience of description, the drawings only show some but not all structures related to the present application. Based on the embodiments in the present application, all other embodiments obtained by those of ordinary skill in the art without creative efforts shall fall within the protection scope of the present application.

The terms "comprising" and "including" and any variations thereof in the present application are intended to cover a non-exclusive inclusion. For example, a process, method, system, product or device comprising a series of steps or elements is not limited to the listed steps or elements, but may optionally also include unlisted steps or elements; or, optionally, other steps or units inherent to these processes, methods, products or devices are also included.

Reference herein to an "embodiment" means that a particular feature, structure, or characteristic described in connection with the embodiment can be included in at least one embodiment of the present application. The appearances of the phrase in various places in the specification are not necessarily all referring to the same embodiment, nor a separate or alternative embodiment that is mutually exclusive of other embodiments. It is explicitly and implicitly understood by those skilled in the art that the embodiments described herein may be combined with other embodiments.

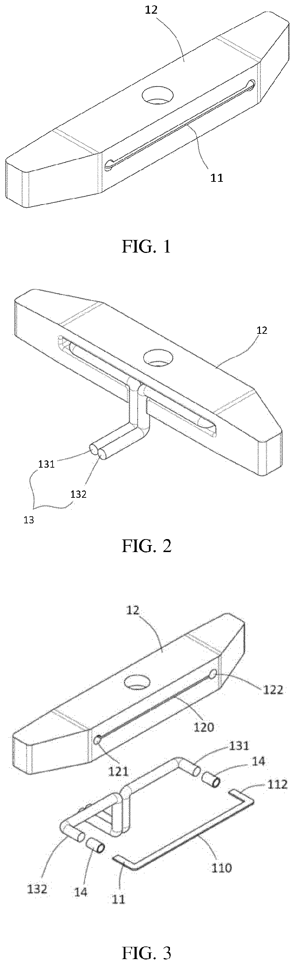

As shown in FIGS. 1 to 5, the present application provides a hot melting structure, including: an insulating seat 12 with a slot 120 arranged along a length direction, and a hot melting element 11 held in the slot 120. One side of the hot melting element 11 is received in the slot 120, and the other side extends to the outside of the slot 120. The hot melting element 11 extending to the outside of the slot 120 is formed as a working edge for being in contact with a garbage bag.

Further, the hot melting element 11 is connected to a power supply circuit through wires 13. When the power supply circuit is energized to the hot melting element 11, the hot melting element 11 can generate heat to perform a fusing process on the garbage bag.

The thermal expansion deformation occurs after the hot melting element 11 is heated. Since a hot-melting working section of the hot melting element 11 is held in the slot 120, the slot 120 can limit the position of the hot-melting working section of the hot melting element 11, so that the deformation of the hot melting element 11 held in the slot 120 in the longitudinal direction can be controlled. As a result, the working edge of the hot melting element 11 is kept straight, and the sealing effect of the garbage bag is improved.

In specific application scenarios, a cross-section of the above-mentioned hot melting element 11 may be a regular shape such as a circle, an ellipse, a triangle, and a rectangle. Considering that the slot 120 needs to effectively position the hot melting element 11, and a sheet-shaped hot melting element 11 can be well received in the slot 120, the cross-section of the hot melting element 11 is preferably rectangular.

Specifically, the hot melting element 11 includes an intermediate section 110 and a first extension section 111 and a second extension section 112 which are disposed on two sides of the intermediate section 110, respectively. The intermediate section 110 is the hot-melting working section of the hot melting element 11, and the intermediate section 110 is partially exposed to the slot 120. The first extension section 111 and the second extension section 112 are both connected to the power supply circuit through the wires 13.

More specifically, the first extension section 111 and the wire 13 may be welded together, or may be crimped together by a fixing member 14. The second extension section 112 and the wire 13 may be welded together, or may be crimped together by a fixing member 14. The fixing member 14 may be a tube-type cold-pressed terminal, a clip, or a buckle, or a component capable of fixing the wire, which is not limited in the present application.

In order to enable the intermediate section 110 to be well received in the slot 120, in the present application, included angles are configured between the first extension section 111 and the intermediate section 110, and between the second extension section 112 and the intermediate section 110. This enables the first extension section 111 and the second extension section 112 to be inserted into the insulating seat 12 along a width direction of the insulating seat 12. Correspondingly, in the present application, the insulating seat 12 is provided with a first through hole 121 for inserting the first extension section 111 and a second through hole 122 for inserting the second extension section 112.

Specifically, the above-mentioned included angle may be an acute angle, an obtuse angle, or a right angle.

Further, the wires 13 include a first wire 132 connected to the first extension section 111 and a second wire 131 connected to the second extension section 112; wherein the first wire 132 may be one of a positive lead and a negative lead, and the second wire 131 is the other of the positive lead and the negative lead.

Since the intermediate section 110 is received in the slot 120, and the first extension section 111 and the second extension section 112 are inserted into the insulating seat 12, the hot melting element 11 cannot be stably mounted to the insulating seat 12. Therefore, in the present application, a lead groove 123 for guiding the wires 13 is provided on the insulating seat 12, so that the wires 13 can clamp and fix two ends of the hot melting element 11.

Specifically, the lead groove 123 includes a first groove 1232 and a second groove 1231 which are opened along the length direction of the insulating seat 12. The first groove 1232 communicates with the first through hole 121 for accommodating the first wire 132. The second groove 1231 communicates with the second through hole 122 for accommodating the second wire 131.

The lead groove 123 further includes one or two third grooves 1233 opened along a height direction of the insulating seat 12. The third groove 1233 is communicated with the first groove 1232 and the second groove 1231. When one third groove 1233 is provided, both the first wire 132 and the second wire 131 are accommodated therein. When two third grooves 1233 are provided, the first wire 132 and the second wire 131 are accommodated in the two lead grooves 123, respectively. Based on this, the first wire 132 is drawn out from the first through hole 121, the first groove 1232 and the third groove 1233. The second wire 131 is drawn out from the second through hole 122, the second groove 1231 and the third groove 1233. Therefore, both the first wire 132 and the second wire 131 have at least two bent portions, that is, the lead groove 123 can limit the position of the first wire 132 and the second wire 131. In the above manner, the first wire 132 and the second wire 131 can clamp and fix two ends of the hot melting element 11.

The hot melting structure in the present application can be used in a fusing mechanism. The fusing mechanism includes the aforementioned hot melting structure and a pressing rod (not shown) for pushing the garbage bag to contact the intermediate section 110. Specifically, the pressing rod can be away from or close to the hot melting structure. When the pressing rod presses the garbage bag on the intermediate section 110, the hot melting element 11 can be energized and heated to cut off the garbage bag.

The fusing mechanism in the present application can be applied to an intelligent trash can. The intelligent trash can includes the aforementioned fusing mechanism and a housing (not shown) for installing the fusing mechanism. Specifically, the fusing mechanism is fixedly arranged in the housing, and the pressing rod is movably arranged in the housing.

It can be understood that the above-mentioned specific applications are only examples of the hot melting structure in the present application, and those skilled in the art can make adaptive adjustments according to the actual situation, which will not be repeated here.

In summary, the insulating seat 12 provided with the slot 120 can limit the position of the hot-melting working section of the hot melting element 11 when the hot melting element 11 is energized and heated, so that the working edge of the hot melting element 11 can be kept in a straight line, thereby improving the sealing effect of the garbage bag.

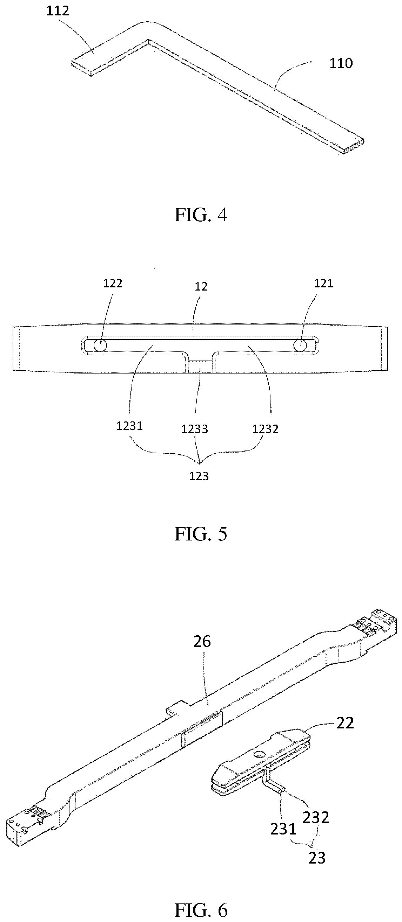

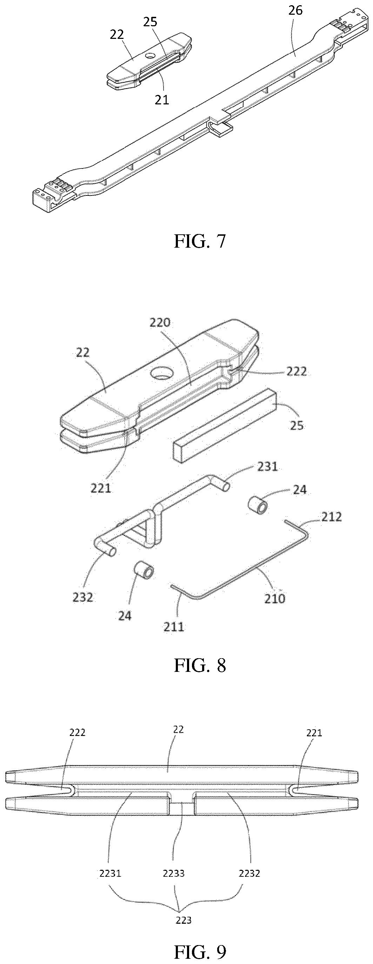

As shown in FIGS. 6 to 9, the present application provides a fusing structure including: an insulating seat 22; a hot melting element 21 disposed on the insulating seat along a length direction of the insulating seat 22; and a pressing rod 26 disposed on a side of the hot melting element 21. The insulating seat 22 defines a slot 220. An insulating elastomer 25 capable of abutting against the hot melting element 21 is received in the slot 220.

It is understandable that the pressing rod 26 can be away from or close to the hot melting element 21. When the pressing rod 26 presses the garbage bag on the hot melting element 21, the insulating elastomer 25 can provide a supporting force to the hot melting element 21, so that the pressing rod 26 can be sufficiently pressed on the hot melting element 21.

Specifically, the insulating elastomer 25 may be made of materials with insulating properties and elastic properties, such as rubber, plastic, and silica gel, which are not limited in the present application.

Furthermore, the hot melting element 21 is connected to a power supply circuit through wires 23. When the power supply circuit is energized to the hot melting element 21, the hot melting element 21 can generate heat to perform a fusing process on the garbage bag.

Specifically, the hot melting element 21 includes an intermediate section 210, and a first extension section 211 and a second extension section 212 disposed on two sides of the intermediate section 210, respectively. Among them, the intermediate section 210 is the hot-melting working section of the hot melting element 21, which is used for fusing the garbage bag and abuts against the insulating elastomer 25. The first extension section 211 and the second extension section 212 are both connected to the power supply circuit through the wires 23.

More specifically, the first extension section 211 and the wire 23 may be welded together, or may be crimped together by a fixing member 24. The second extension section 212 and the wire 23 may be welded together, or may be crimped together by a fixing member 24. The fixing member 24 may be a tube-type cold-pressed terminal, a clip or a buckle, or other component capable of fixing the wire, which is not limited in the present application.

In order to make the intermediate section 210 press against the insulating elastomer 25, an included angle is configured between the first extension section 211 and the intermediate section 210, and between the second extension section 212 and the intermediate section 210 in the present application. This enables the first extension section 211 and the second extension section 212 to extend along a width direction of the insulating seat 22. Correspondingly, in the present application, the insulating seat 22 is provided with a first side groove 221 for accommodating the first extension section 211 and a second side groove 222 for accommodating the second extension section 212.

Specifically, the above-mentioned included angle may be an acute angle, an obtuse angle, or a right angle.

As an embodiment of the present application, the wires 23 include a first wire 232 connected to the first extension section 211 and a second wire 231 connected to the second extension section 212. The first wire 232 is one of the positive lead and the negative lead, and the second wire 231 is the other of the positive lead and the negative lead.

Since the intermediate section 210 is pressed against the insulating elastomer 25, the first extension section 211 and the second extension section 212 are received in the insulating seat 22, so that the hot melting element 21 may not be stably installed on the insulating seat 22. Therefore, in the present application, the wires 23 are wound on the insulating seat 22 to clamp and fix two ends of the hot melting element 21.

Specifically, in the present application, lead grooves 223 for guiding the wires 23 are provided on the insulating seat 22. The lead grooves 223 include a first groove 2232 and a second groove 2231 which are opened along the length direction of the insulating seat 22. The first groove 2232 communicates with the first side groove 221 for accommodating the first wire 232. The second groove 2231 communicates with the second side groove 222 for accommodating the second wire 231.

The lead grooves 223 further include one or two third grooves 2233 opened along a height direction of the insulating seat 22. When one third groove 2233 is provided, the third groove 2233 is communicated with the first groove 2232 and the second groove 2231. Both the first wire 232 and the second wire 231 are accommodated in the third groove 2233. When two lead grooves 223 are provided, one of them communicates with the first groove 2232, and the other communicates with the second groove 2231. The first wire 232 and the second wire 231 are accommodated in the two lead grooves 223, respectively. Based on this, the first wire 232 is drawn out from the first side groove 221, the first groove 2232 and the third groove 2233. The second wire 231 is drawn out from the second side groove 222, the second groove 2231 and the third groove 2233. Therefore, both the first wire 232 and the second wire 231 have at least two bent portions. That is, the lead groove 223 can define the position of the first wire 232 and the second wire 231. In the above manner, the first wire 232 and the second wire 231 can clamp and fix two ends of the hot melting element 21.

As an embodiment of the present application, the pressing rod 26 can reciprocate linearly relative to the hot melting element 21 under the action of a motor, a linear drive or other power devices, so as to press the garbage bag on the intermediate section 210.

The fusing structure in the present application can be used in a trash can. The trash can includes the aforementioned fusing structure and a housing (not shown) for installing the fusing structure. Specifically, the insulating seat 22 is fixedly disposed in the housing. The pressing rod is movably disposed in the housing under the action of the power device.

It is understandable that the above specific applications are only examples of the fusing structure in the present application, and those skilled in the art can make adaptive adjustments according to the actual situation, which will not be repeated here.

In summary, when the pressing rod 26 presses the garbage bag on the hot melting element 21, the hot melting element 21 can be energized and heated to melt the garbage bag. At this time, the insulating elastomer 25 disposed between the insulating seat 22 and the hot melting element 21 can provide a supporting force to the hot melting element 21, so that the garbage bag and the hot melting element 21 can be fully contacted, thereby optimizing the fusing process of the garbage bag.

In the prior art, there is a situation where the pressing rod and the synchronous belt are arranged side by side in a closing and packing mechanism. In this case, the closing and packing mechanism has the defect of occupying a large amount of space for the garbage throwing port, resulting in a smaller size of the actual garbage throwing port, which affects the larger volume of garbage to be thrown.

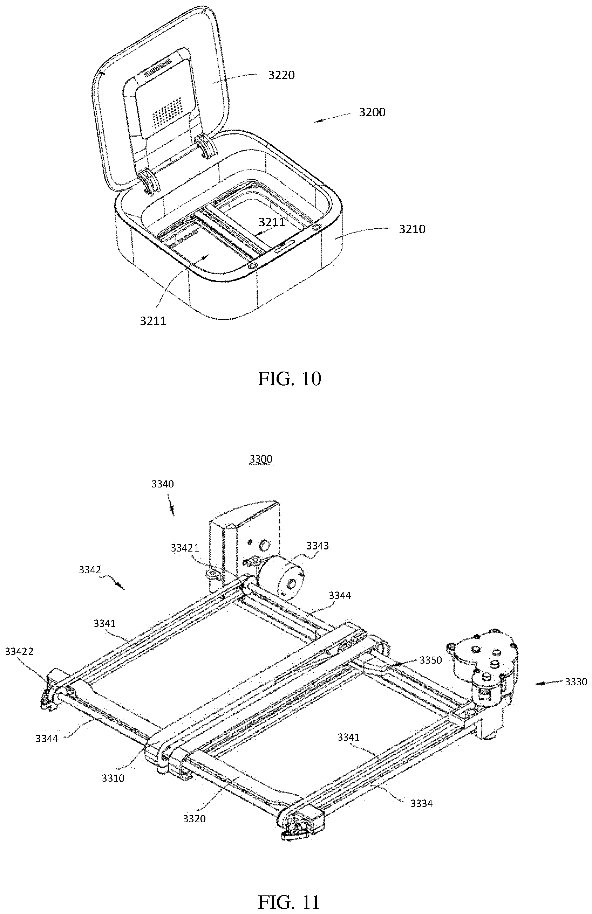

Based on this, the present application provides a closing and packing mechanism, with reference to FIGS. 10 to 16, in the drawings, 3100-barrel body; 3110-barrel mouth; 3200-barrel cover; 3210-large cover; 3211-garbage throwing port; 3220-small cover; 3300-closing and packing mechanism; 3310-first pressing rod; 3311-recess; 33111-opening; 3312-through opening; 3313-convex wing; 3314-protruding portion; 3320-second pressing rod; 3321-pressing block; 3330-first belt transmission unit; 3331-first transmission belt; 3332-first pulley set; 33321-first driving wheel; 33322-first driven wheel; 3333-first drive motor; 3334-guide rod; 3340-second belt transmission unit; 3341-second transmission belt; 3342-second pulley set; 33421-second driving wheel; 33422-second driven wheel; 3343-second drive motor; 3344-transmission shaft; 3350-heating wire seat unit.

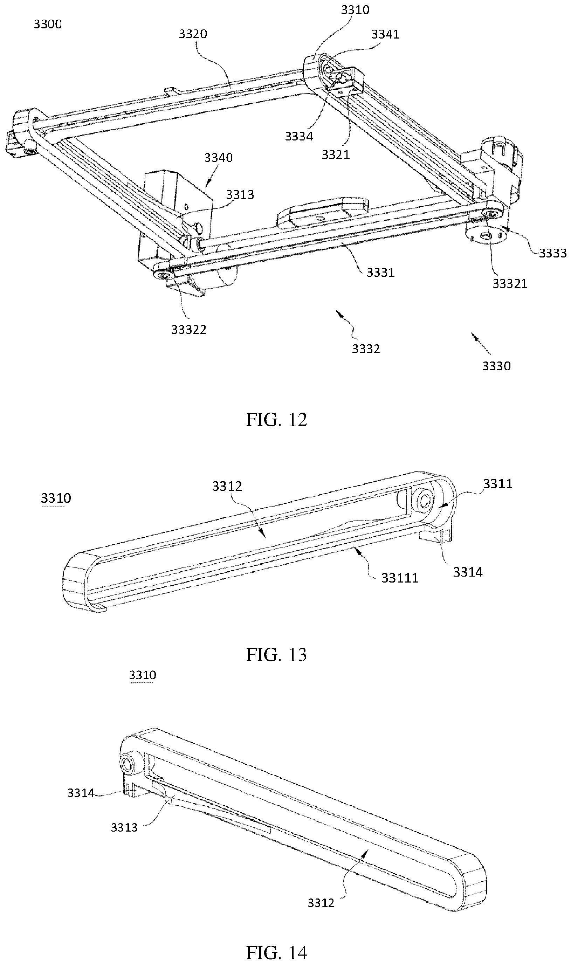

Referring to FIG. 10 to FIG. 12, in which FIG. 10 is a schematic view of a positional relationship between a closing and packing mechanism and a barrel cover proposed by the present application; FIG. 11 is a schematic view of the closing and packing mechanism proposed by the present application in a top view direction; and FIG. 12 is a schematic view of the closing and packing mechanism proposed by the present application in a bottom view direction; in the present application, the closing and packing mechanism is used for automatic closing of a garbage bag, and includes: a pair of first pressing rods 3310 which can move closer synchronously or move away synchronously under the drive of the first belt transmission unit 3330; and a second pressing rod 3320 which is substantially perpendicular to the pair of first pressing rods 3310 and can perform linear reciprocating motion along the length direction of the pair of first pressing rods 3310 under the driving of the second belt transmission unit 3340. The second belt transmission unit 3340 includes a pair of second transmission belts 3341 symmetrically disposed on opposite sides of the pair of first pressure rods 3310 and connected with the second pressing rod 3320. Recesses 3311 are provided on the side walls of the pair of first pressing rods 3310 close to the second transmission belts 3341. The recesses 3311 are configured such that the second transmission belts 3341 can be accommodated in the recesses 3311 when the pair of first pressing rods 3310 are in an extreme position under a status of being synchronously moved away.

In the above manner, the recesses 3311 on the pair of first pressing rods 3310 in the present application can accommodate the second transmission belts 3341 arranged side by side with the first pressing rods 3310, thereby effectively utilizing the space.

It is considered that the second transmission belt 3341 has a tension side and a slack side during operation. Referring to FIG. 13 and FIG. 14, in which FIG. 13 is a schematic structural view of the first pressing rod in FIG. 11 from a viewing angle; and FIG. 14 is a schematic structural view of the first pressing rod in FIG. 11 from another viewing angle; in the present application, the slack side of the second transmission belt 3341 is located at a lower part thereof. In view of this, a bottom groove wall of the recess 3311 is provided with openings 33111 distributed along a length direction thereof. The opening 33111 is configured to form an escape space which can escape a slack side belt body of the second transmission belt 3341, so as to avoid interference between the second transmission belt 3341 and the first pressing rod 3310. Therefore, it has the advantages of simple structure and convenient use.

In order to obtain a better closing effect of the garbage bag, referring to FIG. 11 in conjunction with FIG. 14, the sidewalls of the pair of first pressing rods 3310 are formed with through openings 3312 extending horizontally therethrough. The through openings 3312 are distributed along the length direction of the pair of first pressing rods 3310. The second pressure rod 3320 is disposed through the through opening 3312, and two ends of the second pressure rod 3320 are respectively connected to the second transmission belt 3341. Therefore, the pair of first pressing rods 3310 and the second pressing rod 3320 are basically located on the same plane, so the closing of the garbage bag is relatively flat.

In order to facilitate the closing of the garbage bag, convex wings 3313 are provided on the side walls of the pair of first pressing rods 3310 away from the second transmission belt 3341. The convex wings 3313 can assist in closing the mouth of the garbage bag. The convex wings 3313 are horizontally arranged blocks. The side wall of the convex wing 3313 in contact with the garbage bag is convex in the middle and concave on two sides. Specifically, the convex wings 3313 include a first convex wing provided on one first pressing rod 3310 and a second convex wing provided on the other first pressing rod 3310. The first convex wing and the second convex wing are staggered, in which the first convex wing may be located directly above the second convex wing, or the first convex wing may be located directly under the second convex wing.

Further, the first belt transmission unit 3330 includes a first transmission belt 3331 connected with a pair of first pressing rods 3310, a first pulley set 3332 with the first transmission belt 3331 tensioned, a first drive motor 3333 driving the first pulley set 3332 to move, and a guide rod 3334 parallel to the pair of first pressing rods 3310 for guiding the second pressing rod 3320. An output shaft of the first drive motor 3333 is connected with a first speed reducer. The first drive motor 3333 transmits the motion to the first pulley set 3332 through the first speed reducer. The first pulley set 3332 includes a first driving wheel 33321 and a first driven wheel 33322. Both ends of the first transmission belt 3331 are sleeved on the first driving wheel 33321 and the first driven wheel 33322, respectively, thereby driving the pair of first pressing rods 3310 to move closer synchronously or move away synchronously. The bottoms of the pair of first pressing rods 3310 are formed with protruding portions 3314 extending toward the direction of the first transmission belt 3331. The protruding portion 3314 is provided with a slot. The first transmission belt 3331 is tightly clamped to the above-mentioned slot, thereby driving the pair of first pressing rods 3310 to move synchronously. Specifically, the guide rod 3334 is located on an outside of the second transmission belt 3341. The above-mentioned outside refers to a direction away from the first pressing rod 3310. An end of the second pressing rod 3320 is sleeved on the guide rod 3334 after passing through the second transmission belt 3341. A pressing block 3321 is detachably provided on the end of the second pressing rod 3320. The second transmission belt 3341 is partially held between the pressing block 3321 and the end of the second pressing rod 3320, thereby driving the second pressing rod 3320 to move. The pressing block 3321 is also formed with a lower arc groove. The end of the second pressing rod 3320 is formed with an upper arc groove matched with the lower arc groove. The lower arc groove and the upper arc groove together form a circular hole for the guide rod 3334 to pass through.

Further, the second belt transmission unit 3340 further includes a pair of second pulley sets 3342 tensioned with the second transmission belts 3341, and a second drive motor 3343 driving the pair of second pulley sets 3342 to move. An output shaft of the second drive motor 3343 is connected with a second speed reducer. The second drive motor 3343 transmits the motion to the second pulley set 3342 through the second speed reducer, thereby driving the second transmission belt 3341 to move. Specifically, each second pulley set 3342 includes a second driving wheel 33421 and a second driven wheel 33422. A pair of second driving wheels 33421 are connected by a transmission shaft, and a pair of second driven wheels 33422 are connected by a transmission shaft 3344. The two ends of the pair of first pressing rods 3310 are sleeved on the pair of transmission shafts 3344, respectively, to realize the guiding of the pair of first pressing rods 3310.

It can be understood that the closing and packing mechanism in the present application can be applied to different usage scenarios, and the following examples are used for description.

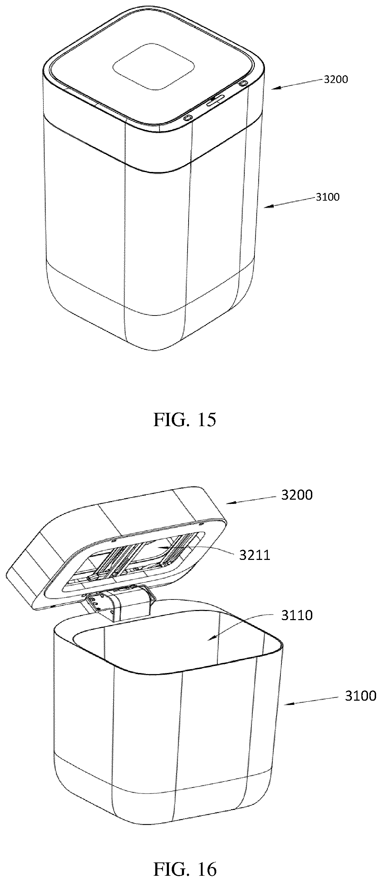

The closing and packing mechanism in the present application can be applied to an intelligent trash can. Referring to FIG. 15 and FIG. 16, in which FIG. 15 is a structural schematic view of an intelligent trash can proposed by the present application; and FIG. 16 is a schematic view when the barrel cover in FIG. 15 is in an open state; the intelligent trash can includes a barrel body 3100 with a hollow interior and an open top to form a cavity for accommodating garbage and a barrel mouth 3110 at the top of the cavity; a barrel cover 3200 pivotally connected to the barrel mouth 3110, the barrel cover 3200 including a large cover 3210 formed with a garbage throwing port 3211 and pivotally connected to the barrel mouth 3110, and a small cover 3220 pivotally connected to the large cover 3210 and capable of covering the garbage throwing port 3211 in a closed state; and a closing and packing mechanism 3300 provided on the large cover 3210. The large cover 3210 is also provided with a heating wire seat unit 3350. The heating wire seat unit 3350 is substantially perpendicular to the pair of first pressing rods 3310 and is disposed opposite to the second pressing rod 3320. The heating wire seat unit 3350 is configured to fuse and seal the closure of the garbage bag. During the working process, the pair of first pressing rods 3310 are driven by the first belt transmission unit 3330 to move closer to the heating wire seat unit 3350 or move away from the heating wire seat unit 3350 synchronously. The second pressing rod 3320 is driven by the second belt transmission unit 3340 to be close to the heating wire seat unit 3350 or away from the heating wire seat unit 3350. When the pair of first pressing rods 3310 are in the extreme position under a status of being synchronously moved away, the second transmission belt 3341 can be accommodated in the recess 3311. Thereby, a situation in which the pair of first pressing rods 3310 and the second transmission belt 3341 are arranged side by side can be avoided, and the area of the garbage throwing port 3211 can be effectively enlarged. When the pair of first pressing rods 3310 are in a limit position of synchronously approaching, the second pressing rod 3320 is in the limit position close to the heating wire seat unit 3350. At this time, the mouth of the garbage bag is closed at the heating wire seat unit 3350 to be fused and sealed.

It can be understood that the above-mentioned specific applications are only examples of the closing and packing mechanism in the present application, and those skilled in the art can make adaptive adjustments according to the actual situation, which will not be repeated here.

In summary, the recesses 3311 on the pair of first pressing rods 3310 in the present application can accommodate the second transmission belts 3341 arranged side by side therewith, thereby effectively utilizing the space. Further, the opening 33111 is provided on the bottom groove wall of the recess 3311. The opening 33111 is configured to form an escape space which can escape the slack side belt body of the second transmission belt 3341, so as to avoid interference between the second transmission belt 3341 and the first pressing rod 3310. Further, convex wings 3313 are provided on the side walls of the pair of first pressing rods 3310 away from the second transmission belt 341. The convex wings 3313 can assist in closing the mouth of the garbage bag. Further, the recesses 3311 can avoid the situation where the pair of the first pressing rods 3310 and the second transmission belt 3341 are arranged side by side, thereby effectively enlarging the area of the garbage throwing port 3211.

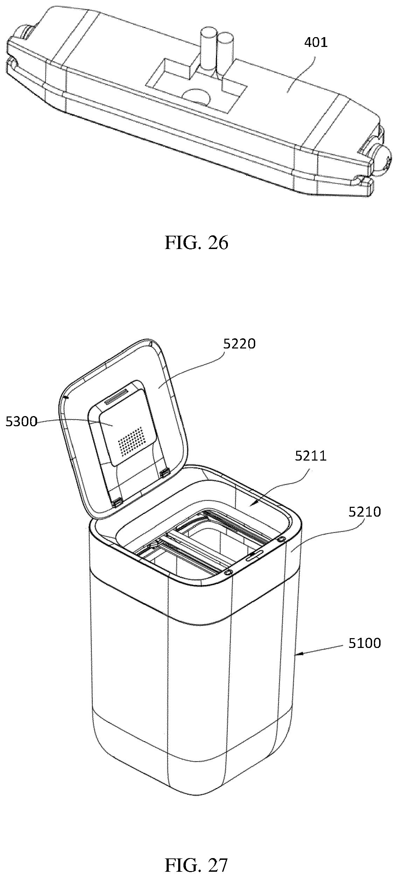

In the prior art, the fusing element on the heating seat of the intelligent trash can is generally arranged as follows: two ends of the fusing element are fastened to a base 401, and an intermediate section is supported on a mounting end surface of the base 401. Referring to FIG. 26, the defect of the above mounting method is as follows: due to the characteristics of thermal expansion and contraction of the heating wire, if the two ends of the heating wire are fixed, the intermediate section of the heating wire is easily deformed due to thermal expansion when it is energized and heated. As a result, its position cannot be fixed, which affects the effect of fuse sealing.

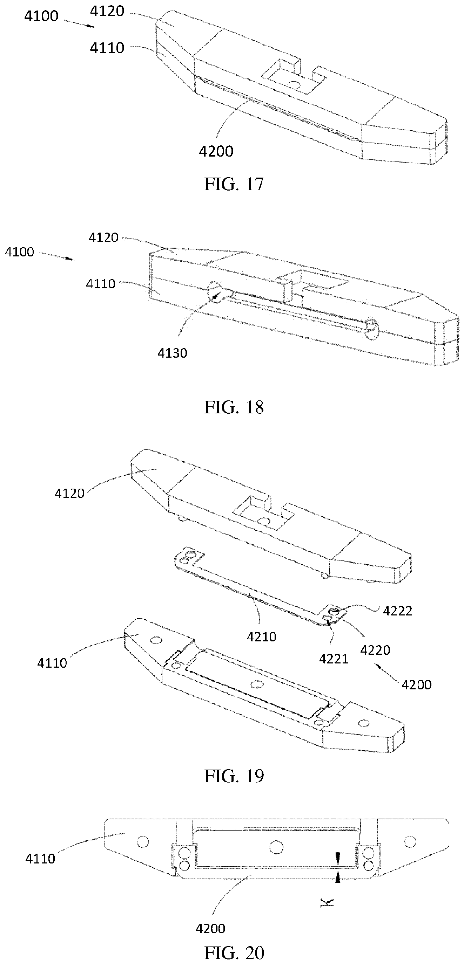

Based on this, the present application provides a heating seat structure. Referring to FIG. 17 to FIG. 19, in which FIG. 17 is a schematic structural view of a heating seat structure described in the present application in a front view direction; FIG. 18 is a structural schematic view of the heating seat structure described in the present application in a rear view direction; FIG. 19 is an exploded structural schematic view of the heating seat structure described in the present application; in the present application, the heating seat structure is applicable to the fusing seal of a garbage bag. The heating seat structure may include: an insulating seat 4100 and a fusing element 4200. The fusing element 4200 is in a sheet shape and is held in the insulating seat 4100. One edge of the fusing element 4200 extends out of the insulating seat 4100 to form a working edge which contacts the garbage bag. The fusing element 4200 includes a fusing element body 4210 of which one edge is a working edge and a connecting piece 4220 formed at opposite ends of the fusing element body 4210.

In the above manner, the fusing element 4200 in the present application is in a sheet shape and is held by the insulating seat 4100. After the fusing element 4200 is electrically connected to a heat melting circuit, it generates heat and undergoes thermal expansion deformation. The insulating seat 4100 can manage and control the thermal expansion and deformation of the fusing element due to electricity and heat generation, and can ensure the position of the intermediate section of the fusing element 4200, so that the working edge of the fusing element 4200 can be kept straight. Thereby, the sealing effect of the garbage bag is effectively improved.

It is understandable that the fusing element 4200 in the present application is provided in a sheet shape, on one hand, the sheet shape is considered to facilitate the clamping of the insulating seat 4100; and on the other hand, the fusing element 4200 with a sheet-like structure has a smaller ability to be deformed by heat than that of the fusing element 4200 with a strip-like structure. That is, the thermal deformation ability of the fusing element 4200 is limited to a certain extent. In the present application, the fusing element 4200 is a sheet-shaped heating wire having protrusions at two ends in a longitudinal direction. The fusing element body 4210 is in a shape of a long strip. The connecting piece 4220 is arranged perpendicular to the length direction of the fusing element body 4210. The dimension of the connecting piece 4220 in the direction perpendicular to the fusing element body 4210 is far smaller than the length dimension of the fusing element body 4210, so as to form the protruding portions at both ends of the fusing element body 4210.

Further, the insulating seat 4100 is provided with a mounting groove. The mounting groove is configured to accommodate the fusing element 4200 and to control the deformation direction of the fusing element 4200 during thermal expansion. The mounting groove is formed with a notch on a side wall of the insulating seat 4100 so that the working edge of the fusing element 4200 is exposed. In this way, it is possible to control the deformation direction of the fusing element 4200, so that the working edge of the fusing element 4200 is kept straight.

It is considered that when the fusing element 4200 is energized and heated, its size will change significantly, and the change in the length direction is especially obvious. Referring to FIG. 20 which is a schematic view of a positional relationship between a lower seat body and the fusing element in the present application, a gap K is preset between the side wall of the fusing element 4200 located in the mounting groove and the groove wall of the mounting groove, so as to form a deformation space. Thereby, the amount of thermal expansion of the fusing element 4200 can be accommodated. It can be understood that the gap K in the length direction of the fusing element 4200 may be larger than the gap K in the width direction of the fusing element 4200; or the gap K in the longitudinal direction and the gap K in the width direction may be equal.

Further, please continue to refer to FIG. 19, the insulating seat 4100 includes an upper seat body 4120 and a lower seat body 4110 which are arranged in an upper part and a lower part. The upper seat body 4120 and the lower seat body 4110 are detachably connected. The upper seat body 4120 and the lower seat body 4110 are made of ceramic material. Considering the convenience of disassembly and assembly, preferably, the upper seat body 4120 and the lower seat body 4110 are connected by bolts. The upper seat body 4120 and the lower seat body 4110 are both provided with through holes for the bolts to pass through. A top surface of the upper seat body 4120 is also provided with a sink groove for accommodating a head of the bolt. Therefore, the connection between the upper seat body 4120 and the lower seat body 4110 is convenient, reliable and stable.

Considering the alignment problem between the upper seat body 4120, the lower seat body 4110 and the fusing element 4200, a pair of positioning posts 4122 extending vertically downward are formed at the bottom of the upper seat body 4120, the top of the lower seat body 4110 is formed with positioning holes 4112 for inserting the positioning posts 4122, and the connecting piece 4220 is provided with mounting holes 4221 for the positioning posts 4122 to pass through. The connecting piece 4220 is a mounting end of the fusing element 4200. Therefore, during the installation process, the mounting positions between the upper seat body 4120, the lower seat body 4110 and the fusing element 4200 can be determined accurately and quickly, which has the advantage of convenient installation.

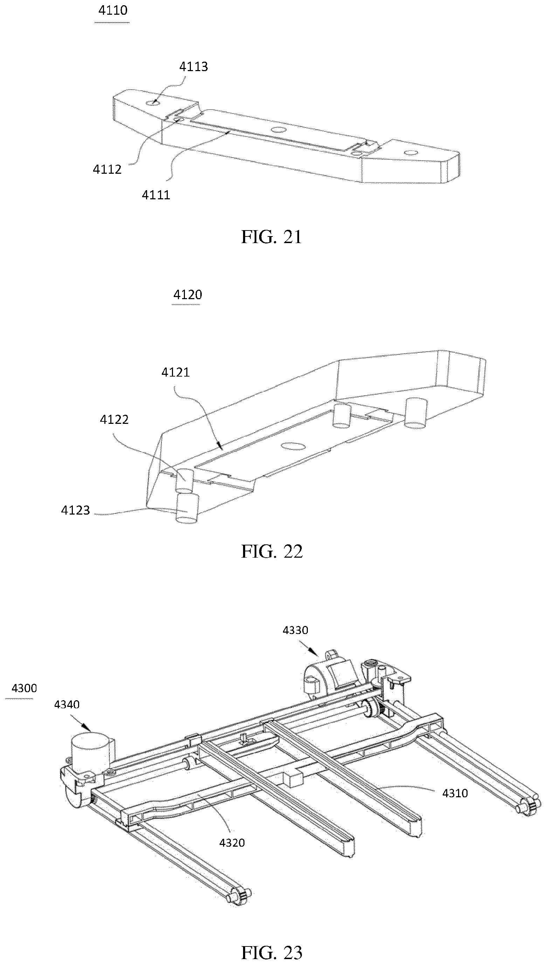

Specifically, referring to FIG. 21 and FIG. 22, in which FIG. 21 is a structural schematic view of the lower seat body in the present application; and FIG. 22 is a structural schematic view of the upper seat body in the present application; in the present application, the mounting groove includes a lower groove body 4111 formed at the top of the lower seat body 4110 and an upper groove body 4121 formed at the bottom of the upper seat body 4120. The positioning posts 4122 are arranged on the upper groove body 4121. The positioning holes 4112 are provided in the lower groove body 4111. Therefore, it has the advantages of convenient production and processing.

Considering the problem of installation and alignment between the upper seat body 4120 and the lower seat body 4110, a pair of limiting posts 4123 extending vertically downward are formed at the bottom of the upper seat body 4120. Limiting holes 4113 for inserting the limiting posts 4123 are formed on the top of the lower seat body 4110. At the same time, in combination with the positioning holes 4112 of the lower seat body 4110 and the positioning posts 4122 of the upper seat body 4120, precise positioning can be achieved, which has the advantage of high installation accuracy.

Further, please continue to refer to FIG. 17 to FIG. 19, the connecting piece 4220 is further provided with connecting holes 4222. The connecting holes 4222 are configured to connect with wires. The insulating seat 4100 is further provided with a wiring hole groove 4130. The wiring hole groove 4130 communicates with the mounting groove. The wiring hole groove 4130 is configured to accommodate the wires.

It can be understood that the heating seat structure in the present application can be applied to different usage scenarios, and the following examples are used for description.

The heating seat structure in the present application can be applied to the closing and fusing mechanism. The closing and fusing mechanism includes the heating seat structure and a pressing rod assembly 4300 for driving the garbage bag to be closed to the heating seat structure.

Referring to FIG. 23 which is the structural schematic view of the closing and fusing mechanism described in the present application, the pressing rod assembly 4300 includes a pair of first pressing rods 4310, a second pressing rod 4320 substantially perpendicular to the first pressing rods 4310, a first driving guide unit 4330 and a second driving guide unit 4340. Specifically, the pair of first pressing rods 4310 are symmetrically distributed on opposite sides of a working edge of the fusing element 4200 and are substantially perpendicular to the working edge of the fusing element 4200. The second pressing rod 4320 is substantially parallel to the working edge of the fusing element 4200. The first driving guide unit 4330 is drivingly connected with the pair of first pressing rods 4310, so as to be able to move closer synchronously or move away synchronously under the driving of the first drive guide unit 4330. The second driving guide unit 4340 is drivingly connected with the second pressing rod 4320, so that under the driving of the second driving guiding unit 4340, the second driving guide unit 4340 can perform linear reciprocating motion along the length direction of the pair of first pressing rods 4310 to approach or move away from the heating seat structure. During operation, the pair of first pressing rods 4310 and the second pressing rod 4320 are driven by the first driving guide unit 4330 and the second driving guide unit 4340, respectively, to move closer to the working edge of the fusing element 4200. Finally, the bag mouth of the garbage bag is closed at the working edge of the fusing element 4200 under the cooperative action of the pair of first pressing rods 4310 and the second pressing rod 4320. After the fusing element 4200 is energized, the bag mouth of the garbage bag is fused and sealed.

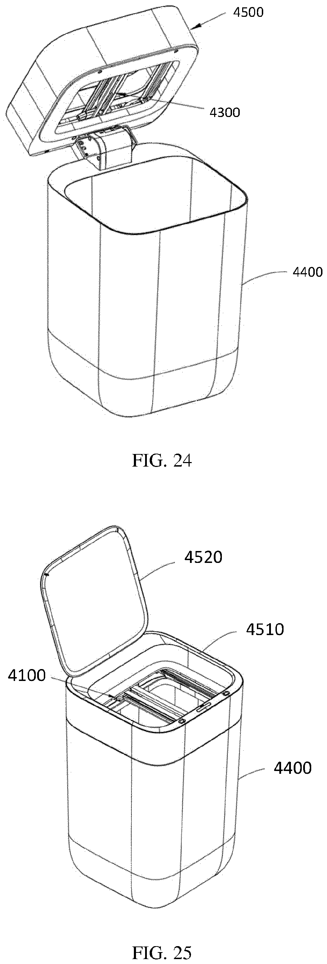

The heating seat structure in the present application is also applicable to an intelligent trash can. Refer to FIG. 24 and FIG. 25, in which FIG. 24 is a first structural schematic view of an intelligent trash can described in the present application; and FIG. 25 is a second structural schematic view of the intelligent trash can described in the application; the intelligent trash can includes a barrel body 4400 with a top opening and a barrel cover 4500 pivotally connected to the top opening of the barrel body 4400. Inside the barrel cover 4500 is an accommodating space for accommodating the closing and fusing mechanism. Specifically, the barrel cover 4500 includes a base cover 4510 formed with a garbage throwing port and a small cover 4520 pivotally connected to the base cover 4510. The closing and fusing mechanism is arranged on the base cover 4510. The base cover 4510 is pivotally connected to the top opening of the barrel body 4400.

It can be understood that the above-mentioned specific applications are only examples of the structure of the heating seat in the present application, and those skilled in the art can make adaptive adjustments according to the actual situation, which will not be repeated here.

In summary, the insulating seat 4100 can control the thermal expansion and deformation of the fusing element due to electricity and heat generation, and can ensure the position of the intermediate section of the fusing element 4200, so that the working edge of the fusing element 4200 remains linear. Further, the positioning posts 4122 and the limiting posts 4123 can accurately and quickly determine the installation positions between the upper seat body 4120, the lower seat body 4110 and the fusing element 4200, which has the advantage of convenient installation.

In the prior art, when throwing garbage into the trash can, especially the intelligent trash can with automatic lid opening function, when the lid is opened, the bacteria in the trash can escape into the air outside the trash can, and people are easily exposed to the bacteria in the trash can.

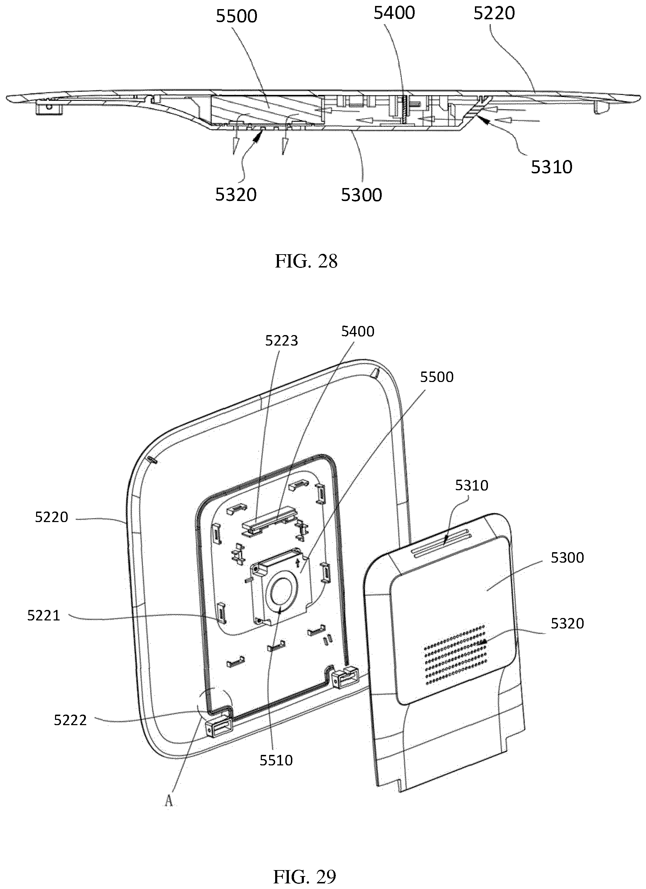

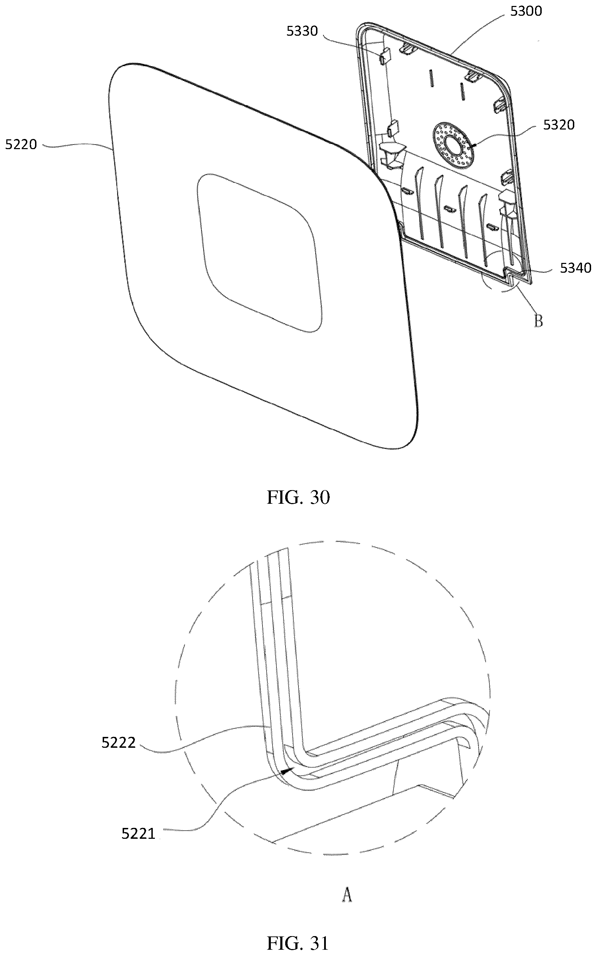

Based on this, the present application provides a sterilization device as shown in FIG. 27 to FIG. 30, in which FIG. 27 is a schematic view of a positional relationship between a sterilization device and a flip cover proposed in the present application; FIG. 28 is a schematic cross-sectional structural view of the sterilization device proposed by the present application; FIG. 29 is an exploded schematic view of the sterilization device proposed by the present application in a front view direction; and FIG. 30 is an exploded schematic view of the sterilization device proposed by the present application in a rear view direction.

In the present application, the sterilization device is applied to a trash can with a flip cover 5220 at the garbage throwing port 5211. The flip cover 5220 is pivotally connected to the garbage throwing port 5211 to form a closed garbage storage space. The sterilization device includes: an ultraviolet sterilization lamp 5400, which is detachably arranged on an inner wall of the flip cover 5220 to generate ultraviolet rays for sterilization; a fan 5500, which is detachably arranged on the inner wall of the flip 5220 so as to circulate the air in the garbage storage space; a cover 5300, which is detachably arranged on the inner wall of the flip cover 5220 and covers the outer periphery of the ultraviolet sterilization lamp 5400 and the fan 5500 to form a sterilization space. The cover 5300 is provided with an air inlet 5310 and an air outlet 5320. A direction from the air inlet 5310 to the air outlet 5320 is an air flow direction in the sterilization space. The ultraviolet sterilization lamp 5400 is located upstream of the air flow direction. The fan 5500 is located downstream in the air flow direction.

Through the above method, the ultraviolet sterilization lamp 5400 in the present application can sterilize the air in the trash can by means of ultraviolet sterilization, which can effectively prevent the growth of bacteria in the trash can and sterilize effectively. The cover 5300 can shield the ultraviolet sterilization lamp 5400 while forming the sterilization space on the inner wall of the flip cover 5220 to prevent the light of the ultraviolet sterilization lamp from directly irradiating the human body. The fan 5500 can promote the circulation of air in the trash can and effectively improve the sterilization effect. Thereby, the user can be prevented from contacting the bacteria in the trash can, which has the advantages of safety and reliability.

Further, the ultraviolet sterilization lamp 5400 is arranged close to the air inlet 5310. Thereby, the ultraviolet-ray can be irradiated to the airflow containing bacteria in time. Ultraviolet irradiation for a long time is beneficial to sterilization. The fan 5500 is positioned close to the air outlet 5320. The air outlet 5510 of the fan 5500 abuts against the air outlet 5320. As a result, the airflow discharged from the fan 5500 can be quickly returned to the trash can body, which effectively improves the circulation efficiency of the fan 5500.

Further, a first snapping structure 5221 is disposed on the inner wall of the flip cover 5220. The cover 5300 is provided with a second snapping structure 5330 which is matched with the first snapping structure 5221. The cover 5300 is snap-fitted to the inner wall of the flip cover 5220 through the cooperation between the second snapping structure 5330 and the first snapping structure 5221. It can be understood that, in addition to the connection manner of snap connection, detachable connection manners such as screw connection and threaded connection can also be used.

Considering the convenience of disassembly and assembly of the cover 5300, the first snapping structure 5221 is a hook, and the second snapping structure 5330 is a slot matched with the hook. Alternatively, the first snapping structure 5221 is a slot, and the second snapping structure 5330 is a hook matched with the slot. The connection method of the hook and the slot has the advantages of simple structure, convenient use, and short disassembly and assembly time.

In order to facilitate the inhalation of the gas containing bacteria in the trash can into the sterilization space surrounded by the cover 5300 and ensure the independence of the sterilization space, the air inlet 5310 is set as a plurality of elongated slots distributed in parallel. That is, the air inlet 5310 consists of a plurality of elongated slots distributed in parallel. In this way, the independence of the sterilization space is ensured under the conditions favorable for the airflow. The term "independence" here means that the sterilization space and the space in the trash can are two independent spaces.

In order to make the air flow discharged from the sterilization space more uniform, the air outlet 5320 is set as a plurality of through holes distributed in a circular array. That is, the air outlet 5320 consists of a plurality of through holes distributed in a circular array. Therefore, the wind speed of the airflow discharged through the air outlet 5320 is relatively uniform, and the garbage in the trash can will not be scattered or disturbed. In the present application, the air outlet 5320 is positioned downward to form a downward pressure to prevent the garbage from "flying".

Further, a sealing structure is provided between the inner wall of the flip cover 5220 and the cover 5300 to improve the air tightness of the sterilization space. Therefore, both the sterilization effect and the circulation speed are ideal.

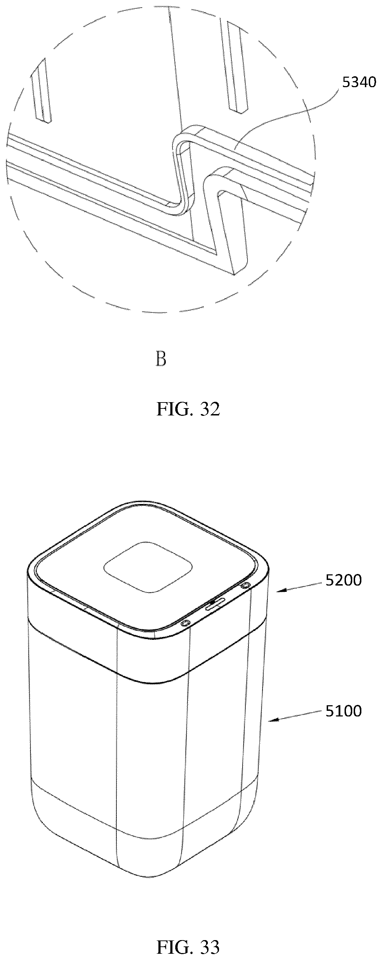

Specifically, referring to FIG. 31 and FIG. 32, in which FIG. 31 is an enlarged structural schematic view of area A in FIG. 29; and FIG. 32 is an enlarged schematic view of region B in FIG. 30, the sealing structure includes a second annular rib 5340 distributed along the edge of the cover 5300 and protruding toward the flip cover 5220, and a first annular rib 5222 formed on the inner wall of the flip cover 5220 and matched with the second annular rib 5340. The second annular rib 5340 and the first annular rib 5222 are both of an integral annular configuration. The first annular rib 5222 is provided with a sealing groove 52221 into which the second annular rib 5340 is inserted. Thereby, the airtightness of the sterilization space can be improved, and the airtightness is good.

Considering the convenience of installation of the ultraviolet sterilization lamp 5400, please continue to refer to FIG. 29, a rib plate 5223 is formed on the inner wall of the flip cover 5220. The rib plate 5223 is configured to enclose a groove body for clamping the ultraviolet sterilization lamp 5400.

Further, both the ultraviolet sterilization lamp 5400 and the fan 5500 are connected to the power supply circuit board through wires to control the opening and closing of the sterilization function. Specifically, when the flip cover 5220 is in a closed state, the sterilization function of the sterilization device is turned on, and the air in the trash can can be sterilized. When the flip cover 5220 is in an open state, the sterilization device is in a closed state. Specifically, when the flip cover 5220 is in the closed state, the sterilization device is activated, which can effectively kill the bacteria in the air in the trash can, and prevent the bacteria in the air of the trash can from escaping into the outside air after the flip cover 5220 is opened. Thus, the user is prevented from contacting the bacteria grown in the trash can, which is more in line with modern people's pursuit of health and hygiene.

It can be understood that the sterilization device in the present application can be applied to different usage scenarios, which will be described with examples below.

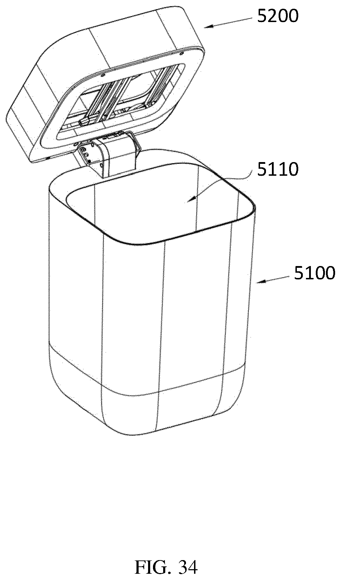

The sterilization device in the present application can be applied to an intelligent trash can. Referring to FIG. 33 and FIG. 34, in which FIG. 33 is a structural schematic view of the intelligent trash can proposed by the present application; and FIG. 34 is a schematic view of the positional relationship between a barrel body and the barrel cover of the intelligent trash can proposed in the present application, the intelligent trash can includes: a barrel body 5100, an interior of which is hollow and the top is open to form a cavity for accommodating garbage and a barrel mouth 5110 at the top of the cavity; a barrel cover 5200, which is pivotally connected to the barrel mouth 5110, the barrel cover 5200 including a barrel cover base 5210 formed with a garbage throwing port 5211 and pivotally connected to the barrel mouth 5110, and a flip cover 5220 pivotally connected to the garbage throwing port 5211; and a sterilization device, which is provided at the flip cover 5220. The sterilization device is the aforementioned sterilization device.

It can be understood that the above-mentioned specific applications are only examples of the sterilization device in the present application, and those skilled in the art can make adaptive adjustments according to the actual situation, which will not be repeated here.

In summary, the ultraviolet sterilization lamp 5400 in the present application can sterilize the air in the trash can by means of ultraviolet sterilization, which can effectively prevent the growth of bacteria in the trash can and sterilize effectively. The cover 5300 can shield the ultraviolet sterilization lamp 5400 while forming the sterilization space on the inner wall of the flip cover 5220 to prevent the light of the ultraviolet sterilization lamp from directly irradiating the human body. The fan 5500 can promote the circulation of air in the trash can and effectively improve the sterilization effect. Further, the air inlet 5310 is set as the plurality of elongated slots distributed in parallel, thereby facilitating the inhalation of the gas containing bacteria in the trash can into the sterilization space enclosed by the cover 5300 while ensuring the independence of the sterilization space. Further, the air outlet 5320 is set as the plurality of through holes distributed in a circular array, thereby making the air flow discharged from the sterilization space relatively uniform.

The number of apparatuses and processing scales described here are intended to simplify the description of the present invention. Applications, modifications and variations to the present invention will be apparent to those skilled in the art.

The above descriptions are merely the embodiments of the present application, and are not intended to limit the patent scope of the present application. All equivalent structures or equivalent process transformations made by using the contents of the description and drawings of the present application, or directly or indirectly applied in other related technical fields, are similarly included in the scope of patent protection of the present application.

## Claims

1. A hot melting structure, comprising:
an insulating seat, the insulating seat defining a slot along a length thereof; and
a hot melting element held in the slot, the hot melting element being partially exposed in the slot;
wherein the hot melting element is connected to a power supply circuit through wires.

2. The hot melting structure according to claim 1, wherein a cross-section of the hot melting element is rectangular.

3. The hot melting structure according to claim 1, wherein the hot melting element comprises:
an intermediate section held in the slot; and
a first extension section and a second extension section which are disposed on two sides of the intermediate section, respectively;
wherein the first extension section and the second extension section are both connected to the power supply circuit through the wire.

4. The hot melting structure according to claim 3, wherein an included angle is formed between the first extension section and the intermediate section, and between the second extension section and the intermediate section.

5. The hot melting structure according to claim 3, wherein the first extension section and the second extension section are inserted into the insulating seat.

6. The hot melting structure according to claim 3, wherein the wires comprise:
a first wire connected to the first extension section; and
a second wire connected to the second extension section.

7. The hot melting structure according to claim 6, wherein the first extension section and the first wire are connected together by a fixing member, and the second extension section and the second wire are connected together by a fixing member.

8. The hot melting structure according to claim 6, wherein a lead groove for accommodating the first wire and the second wire is opened on the insulating seat.

9. A fusing mechanism, comprising the heat melting structure according to any one of claims 1 to 8.

10. An intelligent trash can, comprising a fusing mechanism for fusing a garbage bag; wherein the fusing mechanism is the fusing mechanism according to claim 9.

11. A fusing structure, comprising:
an insulating seat;
a hot melting element disposed on the insulating seat along a length direction of the insulating seat;
an insulating elastomer disposed between the insulating seat and the hot melting element; and
a pressing rod movably disposed on a side of the hot melting element;
wherein the hot melting element is connected to a power supply circuit through wires.

12. The fusing structure according to claim 11, wherein the wires are wound on the insulating seat.

13. The fusing structure according to claim 11, wherein the hot melting element comprises:
an intermediate section abutting against the insulating elastomer; and
a first extension section and a second extension section which are disposed on two sides of the intermediate section, respectively;
wherein the first extension section and the second extension section are both connected to the wires.

14. The fusing structure according to claim 11, wherein an included angle is formed between the first extension section and the intermediate section, and between the second extension section and the intermediate section.

15. The fusing structure according to claim 13, wherein a first side groove and a second side groove are configured on two sides of the insulating seat, respectively; and
the first extension section and the second extension section are received in the first side groove and the second side groove, respectively.

16. The fusing structure according to claim 13, wherein the wires comprise:
a first wire connected to the first extension section; and
a second wire connected to the second extension section.

17. The fusing structure according to claim 16, wherein the first extension section and the first wire are connected together by a fixing member, and the second extension section and the second wire are connected together by a fixing member.

18. The fusing structure according to claim 16, wherein a lead groove for accommodating the first wire and the second wire is opened on the insulating seat.

19. The fusing structure according to claim 18, wherein the lead groove comprises:
a first groove for accommodating the first wire;
a second groove for accommodating the second wire; and
a third groove for accommodating the first wire and the second wire;
wherein the first groove and the second groove are disposed along the length direction of the insulating seat; and the third groove is disposed along a height direction of the insulating seat.

20. A trash can, comprising the fusing structure according to any one of claims 11 to 19.

21. A heating seat structure, applied to a fusing seal of a garbage bag, comprising:
an insulating seat; and
a fusing element, the fusing element being in a shape of a sheet and being held in the insulating seat, a side edge of the fusing element extending out of the insulating seat to form a working edge contacting the garbage bag;
wherein the fusing element comprises a fusing element body of which one side edge is the working edge, and a connecting piece formed on two opposite ends of the fusing element body.

22. The heating seat structure according to claim 21, wherein
the fusing element is a sheet-shaped heating wire having protruding portions at two ends in a longitudinal direction.

23. The heating seat structure according to claim 21, wherein
the insulating seat is provided with a mounting groove;
wherein the mounting groove is formed with a notch on a side wall of the insulating seat so that the working edge of the fusing element is exposed.

24. The heating seat structure according to claim 23, wherein
a gap is preset between a side wall of the fusing element located in the mounting groove and a groove wall of the mounting groove, so as to form a deformation space.

25. The heating seat structure according to claim 23, wherein
the insulating seat comprises an upper seat body and a lower seat body which are disposed in separate upper and lower parts, the upper seat body and the lower seat body being detachably connected;
wherein a bottom of the upper seat body is formed with a pair of positioning posts extending vertically downward, a top of the lower seat body is formed with positioning holes for inserting the positioning posts, and the connecting piece is provided with mounting holes for the positioning posts to pass through.

26. The heating seat structure according to claim 25, wherein
the mounting groove comprises a lower groove body formed on the top of the lower seat body and an upper groove body formed at the bottom of the upper seat body;
wherein the positioning posts are disposed on the upper groove body, and the positioning holes are disposed in the lower groove body.

27. The heating seat structure according to claim 25, wherein
a pair of limiting posts extending vertically downward are formed at the bottom of the upper seat body, and limiting holes are formed on the top of the lower seat body for inserting the limiting posts.

28. The heating seat structure according to claim 21, wherein
the insulating seat is further provided with a wiring hole which is communicated with the mounting groove.

29. A closing and fusing mechanism, comprising the heating seat structure according to any one of claims 21 to 28, and a pressing rod assembly configured to drive the garbage bag to be closed to the heating seat structure.

30. An intelligent trash can, comprising the closing and fusing mechanism according to claim 29.

31. A closing and packing mechanism for automatic closing of a garbage bag, **characterized by** comprising:
a pair of first pressing rods, the pair of first pressing rods configured for being synchronously approached or synchronously moved away under the driving of a first belt transmission unit; and
a second pressing rod, the second pressing rod being perpendicular to the pair of first pressing rods, and the second pressing rod being configured to perform linear reciprocating motion along a length direction of the pair of first pressing rods under the driving of the second belt transmission unit;
wherein the second belt transmission unit comprises a pair of second transmission belts which are symmetrically disposed on opposite sides of the pair of first pressing rods and connected with the second pressing rod; and
wherein recesses are provided on side walls of the pair of first pressing rods adjacent to the second transmission belts, the recesses are configured such that the second transmission belts can be accommodated in the recesses when the pair of first pressing rods are in an extreme position under a status of being synchronously moved away.

32. The closing and packing mechanism according to claim 31, wherein
a bottom groove wall in the recess is provided with openings distributed along a length direction of the recess, and the openings are configured to form an escape space which can escape a slack side belt body of the second transmission belt.

33. The closing and packing mechanism according to claim 31, wherein
sidewalls of the pair of first pressing rods are formed with through openings extending horizontally therethrough, and the through openings are distributed along the length direction of the pair of first pressing rods;
wherein the second pressing rod is disposed through the through openings, and two ends of the second pressing rod are respectively connected to the second transmission belts.

34. The closing and packing mechanism according to claim 31, wherein
the second belt transmission unit further comprises a pair of second pulley sets on which the second transmission belts are tensioned, and a second drive motor which drives the pair of second pulley sets to move.

35. The closing and packing mechanism according to claim 34, wherein
each second pulley set comprises a second driving wheel and a second driven wheel, both the second driving wheels and the second driven wheels are connected by a transmission shaft; wherein two ends of the pair of first pressing rods are sleeved on the pair of transmission shafts, respectively, so as to realize guiding of the pair of first pressing rods.

36. The closing and packing mechanism according to claim 31, wherein
the first belt transmission unit comprises a first transmission belt connected with the pair of first pressing rods, a first pulley set tensioned with the first transmission belt, a first drive motor driving the first pulley set to move, and a guiding rod parallel to the pair of first pressing rods and configured for guiding the second pressing rod.

37. The closing and packing mechanism according to claim 36, wherein
an end of the second pressing rod is sleeved on the guiding rod after passing through the second transmission belt.

38. The closing and packing mechanism according to claim 31, wherein
convex wings are provided on the side walls of the pair of first pressing rods away from the second transmission belt, the convex wings are configured to assist in closing a mouth of the garbage bag;
wherein the convex wings are horizontally disposed blocks, and a side wall of the convex wing in contact with the garbage bag is convex in a middle and concave on two sides.

39. The closing and packing mechanism according to claim 38, wherein
the convex wings comprise a first convex wing provided on one of the first pressing rods and a second convex wing provided on a remaining one of the first pressing rods;
wherein the first convex wing is located directly above the second convex wing; or
the first convex wing is located directly under the second convex wing.

40. An intelligent trash can, comprising the closing and packing mechanism according to any one of claims 31 to 39.

41. A sterilization device, applicable to a trash can with a flip cover at a garbage throwing port, the flip cover being pivotally connected to the garbage throwing port to form a closed garbage accommodating space, the sterilization device comprising:
an ultraviolet sterilization lamp detachably disposed on an inner wall of the flip cover to generate ultraviolet rays for sterilization;
a fan detachably disposed on the inner wall of the flip cover to circulate the air in the garbage accommodating space; and
a cover detachably disposed on the inner wall of the flip cover, and the cover covering an outer periphery of the ultraviolet sterilization lamp and the fan so as to form a sterilization space;
wherein the cover is provided with an air inlet and an air outlet, a direction from the air inlet to the air outlet is an air flow direction in the sterilization space, the ultraviolet sterilization lamp is located upstream of the air flow direction, and the fan is located downstream of the air flow direction.

42. The sterilization device according to claim 41, wherein
the ultraviolet sterilization lamp is disposed adjacent to the air inlet, and the fan is disposed adjacent to the air outlet; and
wherein an outlet of the fan abuts against the air outlet.

43. The sterilization device according to claim 41, wherein
the inner wall of the flip cover is provided with a first snapping structure, the cover is provided with a second snapping structure matched with the first snapping structure;
wherein the cover is snap-fitted to the inner wall of the flip cover through cooperation between the second snapping structure and the first snapping structure.

44. The sterilization device according to claim 43, wherein
the first snapping structure is a hook, and the second snapping structure is a slot matched with the hook; or
the first snapping structure is a slot, and the second snapping structure is a hook matched with the slot.

45. The sterilization device according to claim 41, wherein
the air inlet consists of a plurality of elongated slots distributed in parallel, and the air outlet consists of a plurality of through holes distributed in a circular array.

46. The sterilization device according to claim 41, wherein
a sealing structure is provided between the inner wall of the flip cover and the cover so as to improve the air tightness of the sterilization space.

47. The sterilization device according to claim 46, wherein
the sealing structure comprises a second annular rib distributed along an edge of the cover and protruding toward the flip cover, and a first annular rib formed on the inner wall of the flip cover and matched with the second annular rib;
wherein the second annular rib and the first annular rib are both of an integral annular configuration, and the first annular rib is provided with a sealing groove for inserting the second annular rib.

48. The sterilization device according to claim 41, wherein
a rib plate is further formed on the inner wall of the flip cover, and the rib plate is configured to enclose a groove to hold the ultraviolet sterilization lamp.

49. The sterilization device according to claim 41, wherein
both the ultraviolet sterilization lamp and the fan are connected with a power supply circuit board through wires.

50. An intelligent trash can, comprising:
a barrel body, an interior of the barrel body being hollow and a top of the barrel body being opened so as to form a cavity for accommodating garbage and a barrel mouth at a top of the cavity;
a barrel cover pivotally connected to the barrel mouth, the barrel cover comprising a barrel cover body formed with a garbage throwing port and pivotally connected to the barrel mouth;
a flip cover pivotally connected to the garbage throwing port; and
a sterilization device disposed on the flip cover;
wherein the sterilization device is the sterilization device according to any one of claims 41 to 49.
